# EUROPEAN PATENT APPLICATION

(11) **EP 0 844 238 A1**
(43) Date of publication of application: **27.05.1998**
(21) Application number: 97308953.5
(22) Date of filing: 07.11.1997
(51) Int. Cl.: C07C 323/52, C07C 319/18

(54) **Process for production of sulfide group-containing mercaptocarboxylic acid and ester thereof**

(30) Priority: 25.11.1996 JP 313948/96
(71) Applicant: NIPPON SHOKUBAI CO., LTD., Chuo-ku, Osaka-shi, Osaka-fu 541 (JP)
(72) Inventor: Ishikawa, Ryuichi, Osaka-shi, Osaka-fu (JP)
(74) Representative: Rees, David Christopher

(57) **Abstract**

To prevent the formation of harmful halogen-containing waste and to improve the productivity of a sulfide group-containing mercarptocarboxylic acid ester.

A process for producing a sulfide group-containing mercarptocarboxylic acid ester, which comprises adding one of thiol groups of a dithiol compound to an acrylic acid ester derivative.

## Description

The present invention relates to a process for producing sulfide group-containing mercarptocarboxylic acid and the ester thereof which are widely used as a polymerization chain transfer agent or the like in an industrial field.

Sulfide group-containing mercarptocarboxylic acid esters are widely used as a polymerization chain transfer agent or the like in an industrial field.

A process for producing a sulfide group-containing mercarptocarboxylic acid ester in which chloroacetic acid ester is reacted with ethane-1,2-dithiol in the presence of triethylamine has been disclosed in Chem. Pharm. Bull., 38, pp. 3035-3041 (1990).

However, the above-mentioned conventional process induces the formation of a large amount of a halogen-containing waste such as hydrogen chloride or the like. Accordingly , an enormous energy is required for the treatment to cope with an environmental pollution. Therefore, it is problematic in that the efficiency is decreased disadvantageously in the industrial viewpoint.

An object of this invention, therefore, is to provide a novel process for the production of sulfide group-containing mercarptocarboxylic acid and the ester thereof.

In order to solve the above-mentioned problems, a process for producing a sulfide group-containing mercarptocarboxylic acid ester as mentioned in claim 1 is characterized in that one of thiol groups of a dithiol compound is added to an acrylic acid ester derivative.

A process for producing a sulfide group-containing mercarptocarboxylic acid ester as mentioned in claim 2 is characterized in that in the process of claim 1, the dithiol compound may be represented by the formula:

HS-R-SH

(wherein R represents a hydrocarbon residue, an ether group-containing hydrocarbon residue or a sulfide group-containing hydrocarbon residue, and the number of carbon atoms contained therein is in the range of 2 to 20).

A process for producing a sulfide group-containing mercarptocarboxylic acid ester as mentioned in claim 3 is characterized in that in the process of claim 1 or 2, the dithioi compound may be represented by the formula: 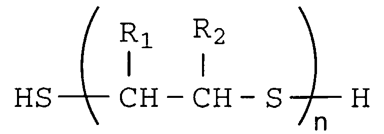 (wherein R₁ and R₂, independently from each other, represent a hydrogen atom or a lower alkyl group, and n is an integer in the range of 1 to 5 which represents a recurring unit).

A process for producing a sulfide group-containing mercarptocarboxylic acid ester as mentioned in claim 4 is characterized in that in the process of any one of claims 1 to 3, the dithiol compound may be used in an amount of at least 1 mol per mol of the acrylic acid ester derivative.

A process for producing a sulfide group-containing mercarptocarboxylic acid ester as mentioned in claim 5 is characterized in that in the process of any one of claims 1 to 4, the reaction may be conducted in the presence of a basic catalyst.

According to the above-mentioned process, a sulfide group-containing mercarptocarboxylic acid ester can be obtained by selectively adding one of the thiol groups of a dithiol compound to an acrylic acid ester derivative such as a (meth)acrylic acid ester. Therefore, no halogen compounds such as chloroacetic acid esters are necessary to be used. As a result, no harmful halogen-containing waste is formed. Thus, an enormous energy for the treatment to cope with the environmental pollution is not required. Consequently, a sulfide group-containing mercarptocarboxylic acid ester can be produced efficiently, and this is industrially advantageous.

A process for producing sulfide group-containing mercarptocarboxylic acid as mentioned in claim 6 comprises hydrolyzing the sulfide group-containing mercarptocarboxylic acid ester obtained by the process of any one of claims 1 to 5.

A process for producing sulfide group-containing mercarptocarboxylic acid as mentioned in claim 7 is characterized in that in the process of claim 6, the sulfide group-containing mercarptocarboxylic acid may be represented by the formula: 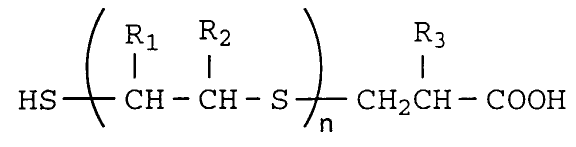 (wherein R₁, R₂ and R₃, independently from each other, represent a hydrogen atom or a lower alkyl group, and n is an integer in the range of 1 to 5 which represents a recurring unit).

One embodiment of this invention will be described as follows.

According to the present invention, a sulfide group-containing mercarptocarboxylic acid ester is produced by adding one of thiol groups of a dithiol compound to an acrylic acid ester derivative.

Although the dithiol compound which is used as a starting material in this invention is not particularly limited, it is preferably represented by the following formula (1):

HS-R-SH (1)

In the above-mentioned formula (1), R represents a hydrocarbon residue, an ether group-containing hydrocarbon residue or a sulfide group-containing hydrocarbon residue, and the number of carbons contained therein is in the range of 2 to 20, preferably 2 to 15. These dithiol compounds maybe used either singly or in combination.

In this invention, although the dithiol compounds wherein in the formula (1), R represents a hydrocarbon residue of 2 to 20 carbon atoms are not particularly limited, they include alkanedithiol compounds such as ethane-1,2-dithiol, propane-1,3-dithiol, propane-1,2-dithiol, butane-1,4-dithiol, and 2,3-dimethylbutane-1,2-dithiol; alicyclic hydrocarbon dithiol compounds such as cyclohexane-1,2-dithiol, and cyclohexane-1,4-dithiol; and aromatic hydrocarbon dithiol compounds such as benzene-1,2-dithiol, and benzene-1,4-dithiol.

Further, in this invention, although the dithiol compounds wherein in the formula (1), R represents an ether group-containing hydrocarbon residue of 2 to 20 carbon atoms are not particularly limited, such compounds as are represented by the following structural formulas;

HS-CH₂CH₂-O-CH₂CH₂-SH

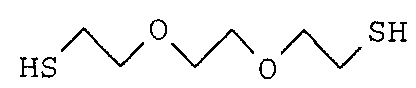

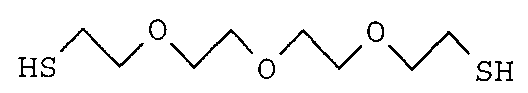

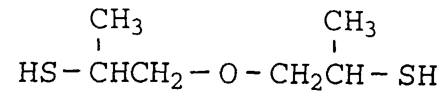

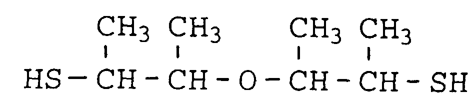

HS-CH₂CH₂CH₂CH₂-O-CH₂CH₂CH₂CH₂-SH

ether group-containing aromatic hydrocarbon dithiol compounds such as 4,4'-dimercaptodiphenyl ether; and ether group-containing alicyclic hydrocarbon dithiol compounds such as 4,4'-dimercaptocyclohexyl ether may be used.

Furthermore, in this invention, although the dithiol compounds wherein in the formula (1), R represents a sulfide group-containing hydrocarbon residue of 2 to 20 carbon atoms are not particularly limited, such compounds as are represented by the following formula (2): 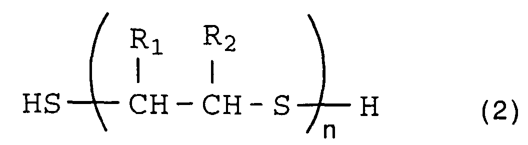 [wherein R₁ and R₂, independently from each other, represent a hydrogen atom or a lower alkyl group, preferably a lower alkyl group of 1 to 4 carbon atoms such as a methyl group, and n is an integer in the range of 1 to 5, preferably 1 to 3 which represents a recurring unit]
may be preferably used.

As typical examples of the compound represented by the above formula (2), compounds represented by the following structural formulas may be cited, for example.

HS-CH₂CH₂-S-CH₂CH₂-SH

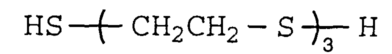

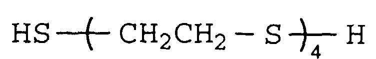

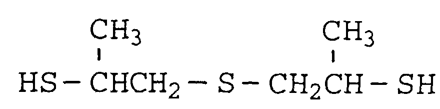

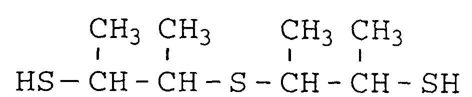

As typical examples of the dithiol compound having a sulfide group-containing hydrocarbon residue of 2 to 20 carbon atoms as mentioned above, besides the compounds represented by the above formula (2), thioether group-containing aliphatic hydrocarbon dithiol compounds represented by the formula:

HS-CH₂CH₂CH₂CH₂-S-CH₂CH₂CH₂CH₂-SH

thioether group-containing aromatic hydrocarbon dithiol compounds such as 4,4'-dimercaptodiphenylthioether, and thioether group-containing alicyclic hydrocarbon dithiol compounds such as 4,4'-dimercaptocyclohexylthioether can be mentioned. However, the dithiol compounds having a sulfide group-containing hydrocarbon residue of 2 to 20 carbon atoms are not critical in the present invention.

Although the acrylic acid ester derivative which is used as a starting material in this invention is not particularly limited, such acrylic acid ester derivatives as represented by the following formula (3):

CH₂ = CR'CO₂R" (3)

[wherein R' represents a hydrogen atom or a lower alkyl group of 1 to 4 carbon atoms, and R" represents a hydrocarbon residue of 1 to 20 carbon atoms]
may be preferably used. These acrylic acid ester derivatives may be used either singly or in combination.

In the above formula (3), R' represents a hydrogen atom or a lower alkyl group of 1 to 4 carbon atoms, and from the industrial availability, an acrylic acid ester in which R' represents a hydrogen atom and a methacrylic acid ester in which R' represents a methyl group maybe preferably used. Further, in the formula (3), R" represents a hydrocarbon residue of 1 to 20 carbon atoms. As typical examples thereof, aliphatic hydrocarbon residues such as methyl, ethyl, propyl, isopropyl, butyl, hexyl, octyl, 2-ethylhexyl, dodecyl and stearyl; alicyclic hydrocarbon residues such as cyclohexyl and alkyl group-substituted cyclohexyl; and aromatic hydrocarbon residues such as phenyl, alkyl group-substituted phenyl and benzyl may be cited.

By using the dithiol compound represented by the formula (1) and the acrylic acid ester derivative represented by the formula (3) as starting materials and adding one of the thiol groups of the dithiol compound to the acrylic acid ester derivative, a sulfide group-containing mercarptocarboxylic acid ester represented by the formula (4):

HS-R-S-CH₂-CHR'-CO₂R" (4)

wherein R, R' and R" have the same meaning as defined above. can be produced.

According to this invention, the sulfide group-containing mercarptocarboxylic acid ester aimed at can be formed more selectively in high yield by reacting the above-mentioned starting materials in the presence of a basic catalyst, which is advantageous. The catalyst is not particularly limited so long as it be basic. Examples of the basic catalyst include amines such as trimethyl amine, triethylamine , and N,N,N',N'-tetramethylethylenediamine; alkali metal hydroxides such as sodium hydroxide, and potassium hydroxide; inorganic solid salts; and anion-exchange resins. In view of the separation of the basic catalyst from the reaction product, the solid bases such as inorganic solid bases and anion-exchange resins are preferable.

As the anion-exchange resins which is preferably used in this invention, various type resins such as weakly basic anion-exchange resins having a tertiary amine structure in the side chain and strongly basic anion-exchange resins having a quaternary ammonium salt structure in the side chain are available. A synthetic resin which is a substrate of the above-mentioned anion-exchange resin is not particularly limited so long as it be insolubilized through cross-linking. It includes polystyrene, polyacrylamide, and an epoxy resin. Specific examples thereof include anion-exchange resins of Amberlite IRA series (made by The Rohm & Haas Company); anion-exchange resins such as of Amberlist A-21, A-26 and A-27 (made by The Rohm & Haas Company); anion-exchange resins such as of Dowex SBR, SAR, and MSR (made by Dow Chemical); and anion-exchange resins of Duolite A series (made by Sumitomo Chemical Co., Ltd.).

Examples of the inorganic solid bases which is preferably used in this invention include alkaline-earth metal oxides such as magnesium oxide, calcium oxide, strontium oxide, and barium oxide; alkaline-earth metal hydroxides such as magnesium hydroxide, and calcium hydroxide; rare-earth metal oxides such as yttrium oxide, and lanthanum oxide; and molecular sieves such as Y-type zeolite which is ion-exchanged with an alkali metal such as sodium, a mordenite-type zeolite, and a ZSM-5-like zeolite. Of these inorganic solid bases, the alkaline-earth metal oxides and/or the alkaline-earth metal hydroxides are preferable.

In this invention, these inorganic solid bases may be used either singly or in combination.

In this invention, the amount of the basic catalyst used may be appropriately determined according to the type of the catalyst. For example, in the case of a homogeneous catalyst such as an amine or the like, the amount of the catalyst is in the range of 0.01 to 10 mol%, preferably 0.1 to 5 mol%, based on the amount of the acrylic acid ester derivative. In the case of the solid basic catalyst, the amount of the catalyst is in the range of 0.01 to 20% by weight, preferably 0.1 to 10% by weight, based on the amount of the acrylic acid ester derivative.

In the present invention, the amount of the dithiol compound to be used is not particularly limited. The dithiol compound is used in an amount which is larger than that of the acrylic acid ester derivative, whereby the acrylic acid ester derivative can be converted into the desired compound in high yield with a high selectivity. However, when the amount of the dithiol compound is too large (for example, 10 or more molar times), no effect corresponding to this amount can be obtained, and the amount of the dithiol compound to be recovered after the completion of the reaction is increased. Thus, it is not economical. On the other hand, when it is extremely small relative to the amount of the acrylic acid ester derivative, a compound in which hydrogen atoms of two thiol groups of the dithiol compound are substituted with acrylic acid ester derivatives (this compound is hereinafter referred to as "a di-addition product") is formed in a large amount, and a yield of a desired compound in which a hydrogen atom of one thiol group is substituted with an acrylic acid ester derivative (this compound is hereinafter referred to as "a mono-addition product") is reduced. By contrast, in the present invention, when the dithiol compound is used in an amount of at least 1 mol, preferably from 1 to 10 mols, more preferably from 1 to 5 mols per mol of the acrylic acid ester derivative, a desired compound is obtained in a yield corresponding to the amount of the dithiol compound added, and it is possible to decrease the yield of the di-addition product and increase the yield of the mono-addition product, which is desirable.

In the present invention, the reaction can be conducted in the absence of a solvent. However, since the reaction of the present invention is an exothermic reaction and the heat has to be removed from the reaction system in the reaction, a solvent may be used to expedite the removal of the heat. Any solvent can be used so long as it be inactive to the reaction and can dissolve the starting materials. Examples thereof include polar, aprotic compounds such as acetonitrile, and N,N-dimethylformamide; ethers such as dioxane, and diethylene glycol dimethyl ether; ketones such as acetone, and methyl ethyl ketone; aliphatic hydrocarbons such as hexane, and cyclohexane; aromatic hydrocarbons such as toluene, and xylene; and halogenated alkyl compounds such as chloroform, and carbon tetrachloride. These may be used either singly or in combination. When the solvent is used, aweight ratio of the solvent to the acrylic acid ester derivative is up to 10, preferably in the range of 1 to 5.

In the present invention, the reaction temperature is not particularly limited. When the reaction temperature is too low, the reaction rate is decreased, and it takes much time for the reaction to proceed. Thus, it is not economical. When the reaction temperature is too high, the side reaction such as polymerization of the acrylic acid ester derivative or the like occurs, decreasing the yield of the final compound. Thus, it is not desirable. The reaction temperature is preferably in the range of 40° to 150 °C, particularly 60° to 100 °C.

The reaction of the acrylic acid ester derivative with the dithiol compound is not particularly limited and can be conducted by any method. For example, the reaction is conducted by charging these two starting materials into a reaction vessel simultaneously, or by successively charging one of these starting materials to the reaction system. In the latter case, it is preferable that the acrylic acid ester derivative be successively added to a larger amount of the dithiol compound, whereby the formation of the di-addition product of the acrylic acid ester derivative to the dithiol compound which is produced by the side reaction can be suppressed and the yield of the desired product and the selectivity therefor can be increased. Further, it is also desirable because the polymerization of the acrylic acid ester derivative can be prevented and the operation be conducted safely. In this successive method, the addition may be conducted either continuously or intermittently.

The above-mentioned reaction according to the present invention may be conducted batchwise, semi-batchwise or continuously. When the reaction is conducted batchwise or semi-batchwise, the acrylic acid ester derivative and the dithiol compound can be fed by any method. In order to prevent the polymerization of the acrylic acid ester derivative, the acrylic acid ester derivative is preferably fed later. Especially, the semi-batchwise reaction is preferable in that the above-mentioned merit of the safety is expected and it is further possible to suppress the formation of the di-addition product and obtain the desired product in higher yield with a higher selectivity. When the continuous reaction is conducted in a continuous tank-type reaction vessel, the catalyst can easily be separated from the reaction solution through a simple procedure such as filtration or the like. When the reaction is conducted in a continuous tube-type reaction vessel filled with a catalyst, a step of separating the catalyst can be eliminated. A desired compound can be obtained by subjecting the reaction solution obtained from an outlet to the distillation, recrystallization, re-precipitation or the like as it is.

In the present invention, the reaction time is not particularly limited. For example, in the case of the batchwise or semi-batchwise reaction, the reaction time is preferably in the range of 1 to 48 hours, particularly 1 to 6 hours, in view of the productivity.

In accordance with the above-mentioned process of the present invention, the sulfide group-containing mercarptocarboxylic acid ester can be obtained in high yield. When this ester is hydrolyzed in the presence of an acid catalyst or a basic catalyst, the corresponding sulfide group-containing mercarptocarboxylic acid can be formed. Especially, a compound represented by formula (5) : 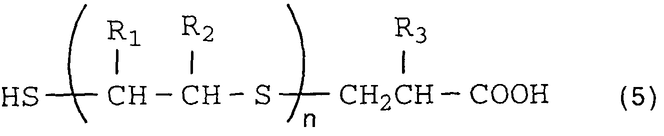 is an useful compound which can be widely used industrially as a polymerization chain transfer agent or the like.

In the above formula (5), R₁ and R₂, independently from each other, have the same meaning as defined in the formula (2), and R₃ represents a hydrogen atom or a lower alkyl group, preferably a lower alkyl group of 1 to 4 carbon atoms such as a methyl group, and n is an integer in the range of 1 to 5, preferably 1 to 3, which represents a recurring unit.

The present invention will be illustrated more specifically by referring to the following working examples. However, the present invention is not limited thereto.

### Example 1

A 300-milliliter four-necked flask fitted with a thermometer, a reflux condenser, a nitrogen introduction tube, a 100-milliliter dropping funnel and a stirrer was charged with 82.50 g (0.878 mol) of ethane-1,2-dithiol as a dithiol compound, 80 ml of acetone and 1.20 g of Amberlist IRA94S (which had been washed and dried). The mixture was heated to 70° to 72 °C while being stirred, and the reaction was controlled such that this temperature was maintained. Methyl acrylate (37.80 g, 0.440 mol) as an acrylic acid ester derivative was drip-fed through the dropping funnel over a period of 1.5 hours, and then the stirring was further continued for 1.5 hours.

After the completion of the reaction, the product was analyzed through gas chromatography. As a result, it was found that the conversion ratio of methyl acrylate was 100% and methyl 6-mercapto-4-thiahexanoate, a desired sulfide group-containing mercarptocarboxylic acid ester, was obtained in a yield of 88.9 mol%, based on methyl acrylate. The results are shown in Table 1.

Subsequently, the above-obtained reaction mixture was separated through distillation at 110° to 111 °C (8 mmHg), and consequently methyl 6-mercapto-4-thiahexanoate represented by the formula:

HS-CH₂CH₂-S-CH₂CH₂-CO₂CH₃

was isolated.

This compound was then hydrolyzed by the following method to obtain 6-mercapto-4-thiahexanoic acid represented by the formula:

HS-CH₂CH₂-S-CH₂CH₂-CO₂H

That is, a 300-milliliter four-necked flask fitted with a packed column having a reflux device, a thermometer and a stirrer was charged with 54 g (0.3 mol, molecular weight 180) of methyl 6-mercapto-4-thiahexanoate, 180 g (10 mols) of water and 5.4 g of a strongly acidic ion-exchange resin (Dowex 650-H) as a catalyst. The reaction was conducted in a nitrogen atmosphere at a reaction temperature of 95° to 100 °C for 6 hours while methanol was distilled off together with a part of water.

Methanol was not distilled off at the initial stage of the reaction. However, as the reaction proceeded, methanol was gradually distilled off.

After the completion of the reaction, the resulting reaction mixture was analyzed. Consequently, it was found that this reaction mixture contained 2.7 g of methyl 6-mercapto-4-thiahexanoate as the starting material and 46.8 g of 6-mercapto-4-thiahexanoic acid. This amount of the final compound corresponds to a reaction rate of 95% based on the starting material and to a yield of 94%.

The types and the amounts of the reaction substrates, the solvent, the catalyst, the reaction conditions and the results are collectively shown in Table 1.

### Examples 2 to 8

The reaction was conducted in the same manner as in Example 1 except that the types and the amounts of the reaction substrates, the solvent, the catalyst and the reaction conditions were changed as shown in Tables 1 and 2. In these Tables, the substrates a and b are represented by the following formulas.

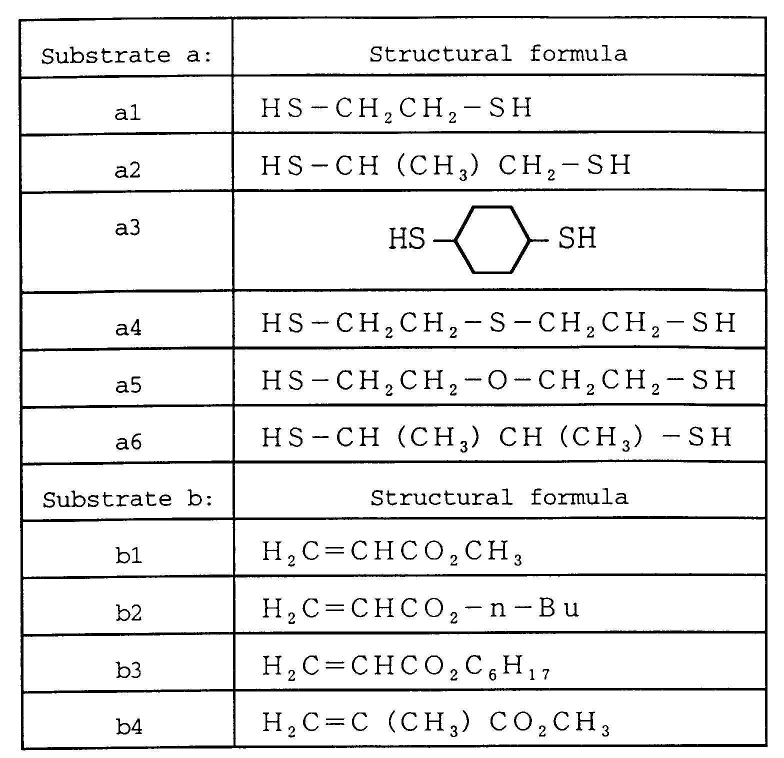

Further, in the Tables 1 and 2, "Amber", "MgO" and "NEt₃" refer to Amberlist IRA 94S, magnesium oxide and triethylamine, respectively. Still further, the conversion ratio (%) is that of an acrylic acid ester derivative.

**Table 1**

| Example | 1 | 2 | 3 | 4 |
|---|---|---|---|---|
| Substrate a | a1 | a1 | a1 | a2 |
| (g) | 82.5 | 72.0 | 72.0 | 44.4 |
| (mols) | 0.878 | 1.20 | 1.20 | 0.60 |
| Substrate b | b1 | b2 | b2 | b3 |
| (g) | 37.8 | 38.4 | 25.8 | 55.2 |
| (mols) | 0.440 | 0.30 | 0.30 | 0.30 |
| a/b molar ratio | 2.0 | 4.0 | 4.0 | 2.0 |
| Solvent | Acetone | Toluene | None | Toluene |
| (ml) | 80 | 80 | | 80 |
| Catalyst | Amber | MgO | Amber | NEt₃ |
| (g) | 1.20 | 3.0 | 1.20 | 0.34 |
| Reaction time (hr) | | | | |
| Drip-feeding | 1.5 | 1.0 | 1.0 | 1.0 |
| Aging | 1.5 | 2.0 | 2.0 | 2.0 |
| Temperature (°C) | 70-72 | 90 | 75 | 75 |
| Conversion (%) | 100 | 100 | 100 | 100 |
| Yield (mol%) | 88.9 | 93.0 | 96.9 | 87 |

**Table 2**

| Example | 5 | 6 | 7 | 8 |
|---|---|---|---|---|
| Substrate a | a3 | a4 | a5 | a6 |
| (g) | 88.8 | 92.4 | 82.8 | 58.8 |
| (mols) | 0.60 | 0.60 | 0.60 | 0.60 |
| Substrate b | b3 | b1 | b1 | b4 |
| (g) | 25.8 | 25.8 | 25.8 | 30.0 |
| (mols) | 0.30 | 0.30 | 0.30 | 0.30 |
| a/b molar ratio | 2.0 | 2.0 | 2.0 | 2.0 |
| Solvent | Toluene | Toluene | Toluene | Toluene |
| (ml) | 80 | 80 | 80 | 80 |
| Catalyst | Amber | Amber | Amber | Amber |
| (g) | 1.20 | 1.20 | 1.20 | 1.20 |
| Reaction time (hr) | | | | |
| Drip-feeding | 1.0 | 1.0 | 1.0 | 1.0 |
| Aging | 2.0 | 2.0 | 2.0 | 4.0 |
| Temperature (°C) | 90 | 70 | 70 | 100 |
| Conversion (%) | 98 | 100 | 100 | 95 |
| Yield (mol%) | 86 | 88 | 89.5 | 83.3 |

As is apparent from Tables 1 and 2, the sulfide group-containing mercarptocarboxylic acid ester was obtained in high yield with a high selectivity in Examples 1 to 8.

As stated above, the process for producing the sulfide group-containing mercarptocarboxylic acid ester as mentioned in claim 1 is a process which comprises adding one of thiol groups of a dithiol compound to an acrylic acid ester derivative.

The process for producing the sulfide group-containing mercarptocarboxylic acid ester of claim 2 is a process in which in the process of claim 1, the dithiol compound may be represented by the formula:

HS-R-SH

(wherein R represents a hydrocarbon residue, an ether group-containing hydrocarbon residue or a sulfide group-containing hydrocarbon residue, and the number of carbon atoms contained therein is in the range of 2 to 20).

The process for producing the sulfide group-containing mercarptocarboxylic acid ester of claim 3 is a process in which in the process of claim 1 or 2, the dithiol compound may be represented by the formula: 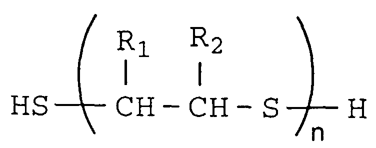 (wherein R₁ and R₂, independently from each other, represent a hydrogen atom or a lower alkyl group, and n is an integer in the range of 1 to 5 which represents a recurring unit).

The process for producing the sulfide group-containing mercarptocarboxylic acid ester of claim 4 is a process in which in the process of any one of claims 1 to 3, the dithiol compound may be used in an amount of at least 1 mol per mol of the acrylic acid ester derivative.

The process for producing the sulfide group-containing mercarptocarboxylic acid ester of claim 5 is a process in which in any one of claims 1 to 4, the reaction may be conducted in the presence of a basic catalyst.

Therefore, an enormous energy for the treatment to cope with the environmental pollution is not required. Consequently, the sulfide group-containing mercarptocarboxylic acid ester can be produced efficiently, and this is industrially advantageous.

The process for producing sulfide group-containing mercarptocarboxylic acid as mentioned in claim 6 comprises hydrolyzing the sulfide group-containing mercarptocarboxylic acid ester obtained by the process of any one of claims 1 to 5.

The process for producing sulfide group-containing mercarptocarboxylic acid of claim 7 is a process in which in the process of claim 6, the sulfide group-containing mercarptocarboxylic acid may be represented by the formula: 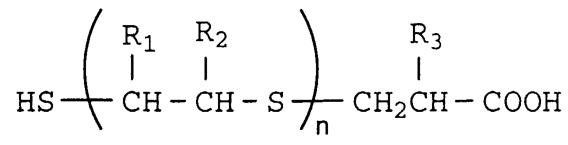 (wherein R₁, R₂ and R₃, independently from each other, represent a hydrogen atom or a lower alkyl group, and n is an integer in the range of 1 to 5 which represents a recurring unit).

Accordingly, a useful compound which is widely used as a polymerization chain transfer agent or the like can be provided.

## Claims

1. A process for producing a sulfide group-containing mercarptocarboxylic acid ester, which comprises adding one of thiol groups of a dithiol compound to an acrylic acid ester derivative.

2. The process of claim 1, wherein said dithiol compound is represented by the formula:
HS-R-SH
wherein R represents a hydrocarbon residue, an ether group-containing hydrocarbon residue or a sulfide group-containing hydrocarbon residue, and the number of carbon atoms contained therein is in the range of 2 to 20.

3. The process of claim 1 or 2, wherein said dithiol compound is represented by the formula: 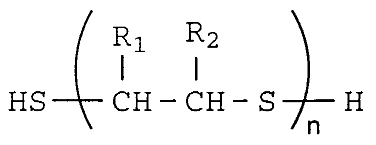 wherein R₁ and R₂, independently from each other, represent a hydrogen atom or a lower alkyl group, and n is an integer in the range of 1 to 5 which represents a recurring unit.

4. The process of any one of claims 1 to 3, wherein said dithiol compound is used in an amount of at least 1 mol per mol of the acrylic acid ester derivative.

5. The process of any one of claims 1 to 4, wherein the reaction is conducted in the presence of a basic catalyst.

6. A process for producing a sulfide group-containing mercarptocarboxylic acid, which comprises hydrolyzing the sulfide group-containing mercarptocarboxylic acid ester obtained by the process of any one of claims 1 to 5.

7. The process of claim 6, wherein said sulfide group-containing mercarptocarboxylic acid is represented by the formula: 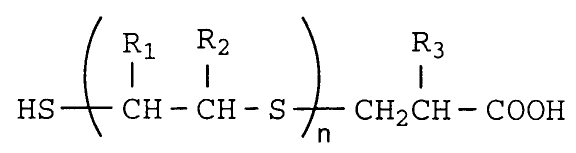 wherein R₁, R₂ and R₃, independently from each other, represent a hydrogen atom or a lower alkyl group, and n is an integer in the range of 1 to 5 which represents a recurring unit.
